# EUROPEAN PATENT SPECIFICATION

(11) **EP 0 578 768 B1**
(45) Date of publication and mention of the grant of the patent: **27.09.1995**
(21) Application number: 92910661.5
(22) Date of filing: 13.03.1992
(51) Int. Cl.: A61K 9/20, A61K 9/00

(54) **INGESTIBLE PHARMACEUTICAL COMPOSITIONS FOR TREATING UPPER GASTROINTESTINAL TRACT DISTRESS**
ESSBARE PHARMAZEUTISCHE ZUSAMMENSETZUNGEN ZUR BEHANDLUNG VON VERDAUUNGSTRAKTSCHMERZEN
COMPOSITIONS PHARMACEUTIQUES ADMINISTREES PAR VOIE BUCCALE DANS LE TRAITEMENT DE TROUBLES DE LA PARTIE SUPERIEURE DES VOIES GASTRO-INTESTINALES

(30) Priority: 04.04.1991 US 680459
(43) Date of publication of application: 19.01.1994
(73) Proprietor: THE PROCTER & GAMBLE COMPANY, Cincinnati, Ohio 45202 (US)
(72) Inventor: UPSON, James Grigg, Springdale, OH 45246 (US); RUSSELL, Carmelita Macklin, Cincinnati, OH 45231 (US)
(74) Representative: Brooks, Maxim Courtney
(86) International application number: US9201981
(87) International publication number: WO9217164

(56) References cited:
- EP-A- 0 080 148
- FR-A- 2 127 011
- US-A- 4 060 091

## Description

The present invention relates to ingestible pharmaceutical compositions comprising pharmaceutical actives useful for treating upper gastrointestinal tract distress and 3-l-menthoxy propane 1,2-diol ("MPD") in amounts effective for providing a cooling sensation in the throat.

Pharmaceutical compositions, such as antacids, useful for treating upper gastrointestinal tract distress (such as heartburn, indigestion, stomachache, etc.) are widely used. They vary depending on the active ingredients, and increasingly differ in the flavors, texture and even forms. The excipients for such compositions are chosen not only as appropriate for the dose form, but also to provide the best possible aesthetics for the compositions, including texture, flavor, after-taste, etc. Depending on the active used, and to some extent the excipients utilized, the time for the therapeutic effect of the active ingredient to be meaningful to the consumer will vary. Frequently, however, the time when the consumer actually begins to receive the benefit of the active ingredient and the time when the consumer perceives the product as starting to "work" are different. Dose form, flavorants, texture, etc. probably all contribute at least in part to this perception. Obviously, for the consumer in need of relief for upper gastrointestinal tract distress, perceiving that the product is working as quickly as possible after ingestion is of great benefit in addition to the true therapeutic relief eventually provided by the active ingredient. FR-A-2,127,011 discloses compositions containing magnesium carbonate, an antacid active, and a 3-substituted p-menthane derivative as cooling agent.

In spite of the large amount of research directed to providing faster acting compositions for treating upper gastrointestinal distress, there continues to be a need for compositions which not only do act faster but also which are perceived by consumers as working faster. Surprisingly, it has been discovered that including the coolant MPD (which is very effective for providing a cooling sensation to the throat) results in the perception by the consumer that the pharmaceutical composition is working. Thus, while this coolant has no apparent therapeutic activity, the signal it provides to the consumer in need of fast relief of upper gastrointestinal distress is of great benefit for the perception that relief is already being provided. Even more surprising is that this improved signal of relief is recognized when compositions according to the present invention are compared against similar compositions containing menthol (which has a noticeably different cooling profile from MPD).

Thus, it is an object of the present invention to provide ingestible pharmaceutical compositions containing a pharmaceutical active useful for treating upper gastrointestinal tract distress (e.g., upset stomach, heartburn, indigestion) which are perceived as providing faster relief and/or greater perceived efficacy and/or longer duration of activity. Furthermore, an object is to provide methods for treating upper gastrointestinal distress by administering pharmaceutical compositions according to the present invention.

These and other objects of the present invention will become readily apparent from the detailed description which follows.

All percentages and ratios used herein are by weight, and all measurements are made at 25°C, unless otherwise specified.

The present invention is directed to ingestible pharmaceutical compositions comprising: (a) a safe and effective amount of a pharmaceutical active useful for treating upper gastrointestinal tract distress; and (b) at least one excipient comprising an amount of 3-l-menthoxy propane 1,2-diol effective for providing a cooling sensation to the throat.

The present invention is also directed to methods for treating upper gastrointestinal tract distress. These methods comprise orally administering to a human patient in need of such treatment a safe and effective amount of a pharmaceutical active useful for treating upper gastrointestinal tract distress and an amount of 3-l-menthoxy propane 1,2-diol effective for providing a cooling sensation to the throat.

The present invention relates to pharmaceutical composition comprising: (a) at least one pharmaceutical active useful for treating upper gastrointestinal tract distress (preferably antacid actives); and (b) at least one excipient comprising 3-l-menthoxy propane 1,2-diol (hereinafter "MPD") effective for providing a cooling sensation to the throat.

Pharmaceutical actives useful for treating upper gastrointestinal tract distress are those materials which are safe and effective when administered orally for treating disorders of the upper gastrointestinal tract (typically the stomach and/or esophagus) which result in symptoms of upper gastrointestinal tract distress (e.g., heartburn, stomachache, indigestion). Such actives include antacid agents and acid secretion prevention agents (e.g., H₂ receptor-blocking antisecretory agents). Antacid agents include, for example, aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, and mixtures thereof. Examples of acid secretion prevention agents include cimetidine, ranitidine, famotidine, omeprazole, and mixtures thereof. Other useful pharmaceutical actives include bismuth-containing agents such as, for example, bismuth subsalicylate, bismuth aluminate, bismuth citrate, bismuth subcitrate, bismuth nitrate, bismuth subcarbonate, bismuth subgalate, and mixtures thereof. A particularly preferred bismuth salt is bismuth subsalicylate.

Preferred for use herein are antacid agents. Preferred antacid agents are aluminum hydroxide, magnesium hydroxide, dihydroxy aluminum sodium carbonate, calcium carbonate, and mixtures thereof. Most preferred is calcium carbonate.

The compositions of the present invention comprise a safe and effective amount of at least one pharmaceutical active useful for treating upper gastrointestinal tract distress. Typically the pharmaceutical active(s) comprise from about 1% to about 99%, by weight, of the pharmaceutical compositions of the present invention, preferably from about 25% to about 60%, and most preferably from about 30% to about 50%.

The pharmaceutical compositions of the present invention also comprise an amount of MPD effective for providing a cooling sensation to the throat. This material is described in detail in US-A-4,459,425, issued July 10, 1984 to Amano et al. While not to be limited by theory, it is believed that the surprising benefits obtained by the use of MPD in the compositions of the present invention are the result of the unique cooling profile for this compound, which is very noticeable in the throat. MPD is commercially available, being sold by Takasago Perfumery Co., Ltd., Tokyo, Japan.

In addition, excipients other than the MPD may optionally be included in the present compositions. The term "excipients", as used herein, means one or more compatible solid or liquid filler diluents or encapsulating substances which are suitable for oral administration to a human. The term "compatible", as used herein, means that the components of the compositions of the present invention are capable of being commingled with the pharmaceutical active, and with each other, in a manner such that there is no interaction which would substantially reduce the pharmaceutical efficacy of the compositions under ordinary use situations. Excipients must, of course, be of sufficiently high purity and sufficiently low toxicity to render them suitable for administration to the human being treated.

Some examples of substances which can serve as excipients in addition to the MPD are sugars such as lactose, glucose and sucrose; starches such as corn-starch and potato starch; cellulose and its derivatives such as sodium carboxymethylcellulose, ethylcellulose, cellulose acetate; powdered tragacanth; malt; gelatin; talc; stearic acid; magnesium stearate; calcium sulfate; vegetable oils such as peanut oil, cottonseed oil, sesame oil, olive oil, corn oil and oil of theobroma; polyols such as propylene glycol, glycerine, sorbitol, mannitol, and polyethylene glycol; agar; and alginic acid; as well as other non-toxic compatible substances used in pharmaceutical formulations. Wetting agents and lubricants such as sodium lauryl sulfate, as well as coloring agents, flavoring agents, sweetening agents (including nonnutritive sweeteners such as aspartame and saccharin), tableting agents, stabilizers, antioxidants, cooling agents, and preservatives, can also be present. Other compatible pharmaceutical additives and actives which are not pharmaceutical actives useful for treating upper gastrointestinal tract distress (e.g., NSAI drugs; pain killers; muscle relaxants) may be included in the compositions of the present invention. Also, it is to be noted that in addition to the MPD, other materials having cooling properties may optionally be included within the excipients, such as menthol, menthol-like compounds such as N-ethyl-p-menthane-3-carboxamide ("WS-3", supplied by Sterling Drugs), and mixtures thereof.

The choice of excipients to be used in conjunction with the pharmaceutical active of the present compositions is basically determined by the dose form for the compositions. The preferred dosage forms are liquid solutions, liquid suspensions, tablets, especially chewable tablets, capsules and the like, comprising a safe and effective amount of the pharmaceutical actives. Excipients suitable for the preparation of dosage forms for oral administration are well-known in the art. Their selection will depend on secondary considerations like taste, cost, shelf stability, which are not critical for the purposes of the present invention, and can be made without difficulty by a person skilled in the art.

The excipients employed in the present ingestible compositions are used at concentrations sufficient to provide a practical size to dosage relationship. Typically, excipients comprise from about 1% to about 99% by weight of the pharmaceutical compositions of the present invention, preferably from about 40% to about 75%, and most preferably from about 50% to about 70%. Additionally, the MPD typically comprises from about 0.01% to about 0.50% by weight of the pharmaceutical compositions of the present invention, preferably from about 0.02% to about 0.20%, and most preferably from about 0.04% to about 0.10%.

The present invention also relates to methods for treating upper gastrointestinal tract distress in humans. These methods comprise orally administering to a human in need of such treatment a safe and effective amount of a pharmaceutical active useful for treating upper gastrointestinal tract distress and an amount of MPD effective for providing a cooling sensation to the throat. Most preferred is administering a safe and effective amount of an ingestible pharmaceutical composition of the present invention.

The following examples further describe and demonstrate embodiments within the scope of the present invention.

### Example I

An ingestible pharmaceutical composition according to the present invention in the form of a chewable antacid tablet is prepared as follows:

| Ingredients | Weight % |
|---|---|
| Granulated calcium carbonate¹⁾ | 42.87% |
| Magnesium stearate | 2.50% |
| Colored speckles | 0.75% |
| Flavorants | 0.78% |
| MPD²⁾ | 0.07% |
| WS-3³⁾ | 0.05% |
| Aspartame | 0.198% |
| Sodium Saccharin | 0.102% |
| Mannitol⁴⁾ | Q.S. |

| | |
|---|---|
| 1) Granulated calcium carbonate containing 93.3% calcium carbonate, 6.3% glucose and 0.4% gelatin; supplied by Whittaker Clark & Daniels, Philadelphia, Pa. | |
| 2) 3-l-menthoxy propane 1,2-diol, supplied by Takasago Perfumery Co., Ltd., Tokyo, Japan. | |
| 3) N-ethyl-p-menthane-3-carboxamide, supplied by Sterling Drugs. | |
| 4) Granulate mannitol supplied by ICI Americas, Inc., Wilmington, Delaware. | |

The above ingredients are dry blended in a mixer until homogeneous, and then direct compressed in a tabletting machine to approximately 8.5 Strong Cobb units hardness to produce chewable antacid tablets each weighing 1.25g (500mg calcium carbonate per tablet).

Ingestion of one or two of these tablets by a human subject suffering from heartburn, acid indigestion and upset stomach associated with these symptoms provides effective relief for this upper gastrointestinal tract distress.

## Claims

1. Ingestible pharmaceutical compositions comprising:
(a) a safe and effective amount of at least one pharmaceutical active useful for treating upper gastrointestinal tract distress; and
(b) at least one excipient comprising an amount of 3-l-menthoxy propane 1,2-diol effective for providing a cooling sensation to the throat.

2. Ingestible pharmaceutical compositions according to Claim 1 wherein the pharmaceutical active is selected from antacid agents, acid secretion prevention agents, bismuth-containing agents, and mixtures thereof.

3. Ingestible pharmaceutical compositions comprising:
(a) from 1% to 99% of at least one pharmaceutical active useful for treating upper gastrointestinal tract distress; and
(b) from 1% to 99% of at least one excipient comprising an amount of 3-l-menthoxy propane 1,2-diol effective for providing a cooling sensation to the throat.

4. Ingestible pharmaceutical compositions according to any of Claims 1-3 wherein the pharmaceutical active is selected from aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate. magnesium glycinate, magnesium hydroxide, magnesium oxide, magnesium trisilicate, sucralfate, cimetidine, ranitidine, famotidine, omeprazole, bismuth subsalicylate, bismuth aluminate, bismuth citrate, bismuth subcitrate, bismuth nitrate, bismuth subcarbonate, bismuth subgalate, and mixtures thereof.

5. Ingestible pharmaceutical compositions according to Claim 4 wherein the excipient further comprises one or more excipients selected from wetting agents, lubricants, tableting agents, flavoring agents, sweetening agents, coloring agents, stabilizers, antioxidants, cooling agents, preservatives, and mixtures thereof.

6. Ingestible pharmaceutical compositions comprising:
(a) from 25% to 60% of at least one pharmaceutical agent useful for treating upper gastrointestinal tract distress selected from aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, cimetidine, ranitidine, famotidine, omeprazole, bismuth subsalicylate, bismuth aluminate, bismuth citrate, bismuth subcitrate, bismuth nitrate, bismuth subcarbonate, bismuth subgalate, and mixtures thereof;
(b) from 40% to 75% of excipients comprising an amount of 3-l-menthoxy propane 1,2-diol effective for providing a cooling sensation to the throat, and at least one other excipient selected from wetting agents, lubricants, coloring agents, flavoring agent, sweetening agents, tableting agents, stabilizers, antioxidants, cooling agents, preservatives, and mixtures thereof.

7. Ingestible pharmaceutical compositions according to Claim 8 wherein the pharmaceutical active is selected from aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, and mixtures thereof, and wherein further the 3-l-menthoxy propane 1,2-diol comprises from 0.01% to 0.50% by weight of the composition.

8. Ingestible pharmaceutical compositions according to any of Claims 1-7 wherein the pharmaceutical active comprises calcium carbonate.

9. Ingestible pharmaceutical compositions according to any of Claims 1-8 wherein the 3-l-menthoxy propane 1,2-diol comprises from 0.02% to 0.20% by weight of the composition.

10. Ingestible pharmaceutical compositions according to any of Claims 1-9 wherein the excipients comprise at least one cooling agent selected from menthol, menthol-like compounds, and mixtures thereof.

11. Compositions according to any of Claims 1-10 comprising the cooling agent N-ethyl-p-menthane-3-carboxamide.

12. Ingestible pharmaceutical compositions according to any of Claims 1-11 in unit dose form.

## Patentansprüche

1. Pharmazeutische Zusammensetzungen zum Einnehmen, welche enthalten:
a) eine sichere und wirksame Menge von wenigstens einem pharmazeutischen Wirkstoff, der zum Behandeln von Schmerzen im oberen Magen-Darm-Trakt nützlich ist; und
b) wenigstens ein Vehikel, welches eine Menge von 3,1-Menthoxypropan-1,2-diol enthält, die zum Hervorrufen eines kühlenden Gefühls im Rachen wirksam ist.

2. Pharmazeutische Zusammensetzungen zum Einnehmen, nach Anspruch 1, wobei der pharmazeutische Wirkstoff aus Antazida-Mitteln, aus Mitteln, welche die Säure-Sekretion verhindern, aus bismuthältigen Mitteln und Gemischen hievon ausgewählt ist.

3. Pharmazeutische Zusammensetzungen zum Einnehmen, welche enthalten:
a) 1 % bis 99 % von wenigstens einem pharmazeutischen Wirkstoff, der zum Behandeln von Schmerzen im oberen Magen-Darm-Trakt nützlich ist; und
b) 1 % bis 99 % von wenigstens einem Vehikel, welches eine Menge von 3,1-Menthoxypropan-1,2-diol enthält, die zum Hervorrufen eines kühlenden Gefühls im Rachen wirksam ist.

4. Pharmazeutische Zusammensetzungen zum Einnehmen, nach einem der Ansprüche 1 bis 3, wobei der pharmazeutische Wirkstoff aus Aluminiumcarbonat, Aluminiumhydroxid, Aluminiumphosphat, Aluminiumhydroxycarbonat, Dihydroxyaluminiumnatriumcarbonat, Aluminiummagnesiumglycinat, Dihydroxyaluminiumaminoacetat, Dihydroxyaluminiumaminoessigsäure, Calciumcarbonat, Calciumphosphat, hydratisierten Aluminiummagnesiumsulfaten, Magnesiumaluminat, Magnesiumaluminosilikaten, Magnesiumcarbonat, Magnesiumglycinat, Magnesiumhydroxid, Magnesiumoxid, Magnesiumtrisilikat, Sucralfat, Cimetidin, Ranitidin, Famotidin, Omeprazol, Bismutsubsalicylat, Bismutaluminat, Bismutcitrat, Bismutsubcitrat, Bismutnitrat, Bismutsubcarbonat, Bismutsubgalat und Gemischen hievon ausgewählt ist.

5. Pharmazeutische Zusammensetzungen zum Einnehmen nach Anspruch 4, wobei das Vehikel weiterhin ein oder mehrere Vehikel umfaßt, die aus Netzmitteln, Schmiermitteln, Tablettierungsmitteln, Geschmacksstoffen, Süßungsmitteln, Färbemitteln, Stabilisatoren, Oxidationsinhibitoren, Kühlmitteln, Konservierungsmitteln und Gemischen hievon ausgewählt sind.

6. Pharmazeutische Zusammensetzungen zum Einnehmen, welche enthalten:
a) 25 % bis 60 % von wenigstens einem pharmazeutischen Mittel, das zum Behandeln von Schmerzen im oberen Magen-Darm-Trakt nützlich ist, und welches aus Aluminiumcarbonat, Aluminiumhydroxid, Aluminiumphosphat, Aluminiumhydroxycarbonat, Dihydroxyaluminiumnatriumcarbonat, Aluminiummagnesiumglycinat, Dihydroxyaluminiumaminoacetat, Dihydroxyaluminiumaminoessigsäure, Calciumcarbonat, Calciumphosphat, hydratisierten Aluminiummagnesiumsulfaten, Magnesiumaluminat, Magnesiumaluminosilikaten, Magnesiumcarbonat, Magnesiumglycinat, Magnesiumhydroxid, Magnesiumoxid, Magnesiumtrisilikat, Sucralfat, Cimetidin, Ranitidin, Famotidin, Omeprazol, Bismutsubsalicylat, Bismutaluminat, Bismutcitrat, Bismutsubcitrat, Bismutnitrat, Bismutsubcarbonat, Bismutsubgalat und Gemischen hievon ausgewählt ist;
b) 40 % bis 75 % von Vehikeln, welche eine Menge an 3,1-Menthoxypropan-1,2-diol enthalten, die zum Hervorrufen eines kühlenden Gefühls im Rachen wirksam ist; und wenigstens ein weiteres Vehikel, das aus Netzmitteln, Schmiermitteln, Färbemitteln, Geschmacksstoffen, Süßungsmitteln, Tablettierungsmitteln, Stabilisatoren, Oxidationsinhibitoren, Kühlmitteln, Konservierungsmitteln und Gemischen hievon ausgewählt ist.

7. Pharmazeutische Zusammensetzungen zum Einnehmen, nach Anspruch 8, wobei der pharmazeutische Wirkstoff aus Aluminiumcarbonat, Aluminiumhydroxid, Aluminiumphosphat, Aluminiumhydroxycarbonat, Dihydroxyaluminiumnatriumcarbonat, Aluminiummagnesiumglycinat, Dihydroxyaluminiumaminoacetat, Dihydroxyaluminiumaminoessigsäure, Calciumcarbonat, Calciumphosphat, hydratisierten Aluminiummagnesiumsulfaten, Magnesiumaluminat, Magnesiumaluminosilikaten, Magnesiumcarbonat, Magnesiumglycinat, Magnesiumhydroxid, Magnesiumoxid, Magnesiumtrisilikat, Sucralfat und Gemischen ausgewählt ist, und wobei weiterhin das 3,1-Menthoxypropan-1,2-diol 0,01 Gew.-% bis 0,50 Gew.-% der Zusammensetzung ausmacht.

8. Pharmazeutische Zusammensetzungen zum Einnehmen nach einem der Ansprüche 1 bis 7, wobei der pharmazeutische Wirkstoff Calciumcarbonat umfaßt.

9. Pharmazeutische Zusammensetzungen zum Einnehmen, nach einem der Ansprüche 1 bis 8, wobei das 3,1-Menthoxypropan-1,2-diol 0,02 Gew.-% bis 0,20 Gew.-% der Zusammensetzung ausmacht.

10. Pharmazeutische Zusammensetzungen zum Einnehmen, nach einem der Ansprüche 1 bis 9, wobei die Vehikel wenigstens ein Kühlmittel umfassen, das aus Menthol, mentholartigen Verbindungen und Gemischen hievon ausgewählt ist.

11. Zusammensetzungen nach einem der Ansprüche 1 bis 10, welche das Kühlmittel N-Ethyl-p-menthan-3-carboxamid enthalten.

12. Pharmazeutische Zusammensetzungen zum Einnehmen, nach einem der Ansprüche 1 bis 11, in Einheitsdosisform.

## Revendications

1. Compositions pharmaceutiques administrées par voie buccale comprenant:
(a) une quantité inoffensive et efficace d'au moins une substance pharmaceutiquement active utile pour traiter les douleurs dans la partie supérieure du tractus gastro-intestinal; et
(b) au moins un excipient comprenant une quantité de 3-l-menthoxypropane-1,2-diol efficace pour procurer une sensation de rafraîchissement dans la gorge.

2. Compositions pharmaceutiques administrées par voie buccale selon la revendication 1, dans lesquelles la substance pharmaceutiquement active est choisie parmi les agents antiacides, les agents de prévention de la sécrétion acide, les agents contenant du bismuth, et les mélanges de ceux-ci.

3. Compositions pharmaceutiques administrées par voie buccale comprenant:
(a) de 1% à 99% d'au moins une substance pharmaceutiquement active utile pour traiter les douleurs dans la partie supérieure du tractus gastro-intestinal; et
(b) de 1% à 99% d'au moins un excipient comprenant une quantité de 3-l-menthoxypropane-1,2-diol efficace pour procurer une sensation de rafraîchissement dans la gorge.

4. Compositions pharmaceutiques administrées par voie buccale selon l'une quelconque des revendications 1-3, dans lesquelles la substance pharmaceutiquement active est choisie parmi le carbonate d'aluminium, l'hydroxyde d'aluminium, le phosphate d'aluminium, l'hydroxycarbonate d'aluminium, le carbonate de dihydroxy-aluminium-sodium, le glycinate d'aluminium-magnésium, l'aminoacétate de dihydroxy-aluminium, l'acide dihydroxy-aluminium-aminoacétique, le carbonate de calcium, le phosphate de calcium, les sulfates d'aluminium-magnésium hydratés, l'aluminate de magnésium, les aluminosilicates de magnésium, le carbonate de magnésium, le glycinate de magnésium, l'hydroxyde de magnésium, l'oxyde de magnésium, le trisilicate de magnésium, le sucralfate, la cimétidine, la ranitidine, la famotidine, l'oméprasole, le sous-salicylate de bismuth, l'aluminate de bismuth, le citrate de bismuth, le sous-citrate de bismuth, le nitrate de bismuth, le sous-carbonate de bismuth, le sous-gallate de bismuth, et les mélanges de ceux-ci.

5. Compositions pharmaceutiques administrées par voie buccale selon la revendication 4, dans lesquelles l'excipient comprend en outre un ou plusieurs excipients choisis parmi les agents mouillants, les lubrifiants, les agents de compression, les arômes, les agents édulcorants, les agents colorants, les stabilisants, les antioxydants, les agents rafraîchissants, les conservateurs, et les mélanges de ceux-ci.

6. Compositions pharmaceutiques administrées par voie buccale comprenant:
(a) de 25% à 60% d'au moins un agent pharmaceutique utile pour traiter les douleurs dans la partie supérieure du tractus gastro-intestinal, choisi parmi le carbonate d'aluminium, l'hydroxyde d'aluminium, le phosphate d'aluminium, l'hydroxycarbonate d'aluminium, le carbonate de dihydroxy-aluminium-sodium, le glycinate d'aluminium-magnésium, l'aminoacétate de dihydroxy-aluminium, l'acide dihydroxy-aluminium-aminoacétique, le carbonate de calcium, le phosphate de calcium, les sulfates d'aluminium-magnésium hydratés, l'aluminate de magnésium, les aluminosilicates de magnésium, le carbonate de magnésium, le glycinate de magnésium, l'hydroxyde de magnésium, l'oxyde de magnésium, le trisilicate de magnésium, le sucralfate, la cimétidine, la ranitidine, la famotidine, l'oméprasole, le sous-salicylate de bismuth, l'aluminate de bismuth, le citrate de bismuth, le sous-citrate de bismuth, le nitrate de bismuth, le sous-carbonate de bismuth, le sous-gallate de bismuth, et les mélanges de ceux-ci;
(b) de 40% à 75% d'excipients comprenant une quantité de 3-l-menthoxypropane-1,2-diol efficace pour procurer une sensation de rafraîchissement dans la gorge, et au moins un autre excipient choisi parmi les agents mouillants, les lubrifiants, les agents colorants, les arômes, les agents édulcorants, les agents de compression, les stabilisants, les antioxydants, les agents rafraîchissants, les conservateurs, et les mélanges de ceux-ci.

7. Compositions pharmaceutiques administrées par voie buccale selon la revendication 6, dans lesquelles la substance pharmaceutique active est choisie parmi le carbonate d'aluminium, l'hydroxyde d'aluminium, le phosphate d'aluminium, l'hydroxycarbonate d'aluminium, le carbonate de dihydroxy-aluminium-sodium, le glycinate d'aluminium-magnésium, l'aminoacétate de dihydroxy-aluminium, l'acide dihydroxy-aluminium-aminoacétique, le carbonate de calcium, le phosphate de calcium, les sulfates d'aluminium-magnésium hydratés, l'aluminate de magnésium, les aluminosilicates de magnésium, le carbonate de magnésium, le glycinate de magnésium, l'hydroxyde de magnésium, l'oxyde de magnésium, le trisilicate de magnésium, le sucralfate, et les mélanges de ceux-ci, et dans lesquelles, en outre, le 3-l-menthoxypropane-1,2-diol constitue de 0,01% à 0,50% en poids de la composition.

8. Compositions pharmaceutiques administrées par voie buccale selon l'une quelconque des revendications 1-7, dans lesquelles la substance pharmaceutique active comprend du carbonate de calcium.

9. Compositions pharmaceutiques administrées par voie buccale selon l'une quelconque des revendications 1-8, dans lesquelles le 3-l-menthoxypropane-1,2-diol constitue de 0,02% à 0,20% en poids de la composition.

10. Compositions pharmaceutiques administrées par voie buccale selon l'une quelconque des revendications 1-9, dans lesquelles les excipients comprennent au moins un agent rafraîchissant choisi parmi le menthol, les composés analogues au menthol, et les mélanges de ceux-ci.

11. Compositions selon l'une quelconque des revendications 1-10 comprenant l'agent rafraîchissant N-éthyl-p-menthane-3-carboxamide.

12. Compositions pharmaceutiques administrées par voie buccale selon l'une quelconque des revendications 1-11 sous forme de dose unitaire.
